Europäisches Patentamt

(19) European Patent Office (11) Publication number: **0 127 297**

Office européen des brevets **B1**

(12) **EUROPEAN PATENT SPECIFICATION**

(45) Date of publication of patent specification: **23.08.89** (51) Int. Cl.⁴: **A 61 K 47/00, A 61 K 45/08**

(21) Application number: **84302509.9**

(22) Date of filing: **12.04.84**

(54) **Vitamin composition with enhanced bioavailability and method of administering same.**

(30) Priority: **02.05.83 AU 9132/83**

(43) Date of publication of application:
**05.12.84 Bulletin 84/49**

(45) Publication of the grant of the patent:
**23.08.89 Bulletin 89/34**

(84) Designated Contracting States:
**DE FR GB IT**

(56) References cited:
**FR-M- 8 014**
**GB-A-1 576 528**

**UNLISTED DRUGS, vol. 13, no. 3, March 1961.**
**CHATMAN, NEW JERSEY (US) page 30b:**
**"Polymul"**

The file contains technical information
submitted after the application was filed and
not included in this specification

(73) Proprietor: **R.P. SCHERER CORPORATION**
**2075 West Big Beaver Road**
**Troy Michigan 48099 (US)**

(72) Inventor: **Bateman, Neil E.**
**23 Edro Avenue**
**East Brighton Melbourne Victoria (AU)**

(74) Representative: **Allen, Oliver John Richard et al**
**Lloyd Wise, Tregear & Co. Norman House**
**105-109 Strand**
**London, WC2R 0AE (GB)**

Courier Press, Leamington Spa, England.

# EP 0 127 297 B1

## Description

The present invention relates to vitamin compositions, particularly vitamin compositions in a solid unit dosage form.

Vitamins are a group of substances of diverse chemical composition. They are in general inorganic and organic substances that must be provided in small quantities in the diet for the synthesis by tissues, of cofactors that are essential for various metabolic reactions. As is well known, vitamin deficiency may be due to improper or inadequate diet or may be caused by a variety of disease conditions. Examples are diseases of the liver and biliary tract, prolonged diarrhoea from any cause, hyperthyrodism, anemia together with a variety of other disorders of the digestive system, including those related to alcoholism. There are therefore available numerous vitamin tablets, capsules and injections to aid in the treatment of such vitamin deficiencies.

It has been the practice to prepare vitamin compositions which contain a single or a plurality of vitamins by including in the composition an oily component, for example, vegetable oil, to solubilise the oil soluble vitamins and a pharmaceutical adjunct such as polyoxyethylene sorbitol monooleate which is said to be favourable to the absorption of the vitamins. Such a composition is disclosed in French Pharmacecutical Patent No. 8.014M.

The effectiveness of the intake of such vitamin compositions is dependent, *inter alia,* upon the level of bioavailability of these vitamins in the patient's system.

Bioavailability is defined as the rate and extent of uptake (absorption) by an animal or human of a chemical substance — either natural or synthetic. Bioavailability may be expressed in percentage terms over a predetermined time versus concentration of drug substance or vitamin in blood plasma. This is plotted as a curve and the area under the curve (AUC) is then measured and can be compared for several dosage forms to measure the bioavailability of a drug or vitamin. An increase in AUC thus indicates an increase in bioavailability.

It would be desirable if the level of bioavailability could be increased as this may have the effect of increasing the effectiveness of the vitamin intake. This may also have the effect of reducing the dosages required.

Accordingly, it is an object of the present invention to overcome, or at least alleviate, one or more of the deficiencies related to the prior art.

Thus there is provided in accordnace with the invention a vitamin composition for incorporation into a solid dosage form comprising a unit dosage amount of at least one vitamin and a primary pharmaceutical adjunct, said vitamin comprising 10 to 80% of the total weight of the composition and said primary pharmaceutical adjunct being a polyoxyethylene sorbitan ester, the ester being 30 to 80% of the total weight of the composition, characterised in that the said composition contains a secondary pharmaceutical adjunct consisting of ethanol, glycerol, sorbitol and/or propylene glycol.

While it is unclear as to the mechanism by which bioavailability is enhanced, it is postulated that the increase in bioavailability could be attributed to presenting the vitamins to the sites of absorption in the gastrointestinal tract in an optimal form. This may be extrapolated to all vitamins as the rate limiting step in absorption and hence bioavailability. The dissolution and presentation of the vitamins to the gastrointestinal tract in optimal physical form will vary. For oil-soluble vitamins, microemulsion would appear to be the optimum physical form. For water-soluble vitamins, solution would appear to be the optimum physical form.

An oil-soluble vitamin component may be selected from vitamin A, D, E, K or a mixture thereof. A water-soluble vitamin component may be selected from vitamin $B_1$, $B_2$, $B_6$, $B_{12}$, C, niacinimide and pantothenic acid or a mixture thereof. The preferred oil soluble vitamins are vitamins A and E.

The vitamins may be utilized in any standard form. For example, vitamin A may be presented as Vitamin A palmitate. Vitamin D may be present as calciferol solution. Vitamin E may be present as d-alpha tocopherol. Vitamins $B_1$ and $B_6$ may be present as their hydrochloride derivatives.

The primary adjunct is from the polyoxyethylene sorbitan ester group, in particular, the sorbitan esters sold under the commercial names of Polysorbate® 20 and Polysorbate® 80. These compounds are listed in the British Pharmacopoeia and are safe for use in food and pharmaceuticals.

The vitamin compositions may include a single vitamin component, for example, the oil-soluble vitamins when used alone may be compounded with a polyoxyethylene sorbitan ester, *e.g.* Polysorbate® 80, together with an alcohol such as ethanol and glycerol. A further sorbitan ester such as sorbitan monooleate may also be included. For such oil-soluble compositions, the amount of vitamin used may vary from approximately 15 to 60% by weight.

The polyoxyethylene sorbitan ester component is desirably present in relatively high quantities for example from approximately 35 to 75% by weight.

The vitamin composition may be of the multi-vitamin type. For example, the vitamin composition may include vitamins A, D, E, $B_1$, $B_2$, $B_6$, $B_{12}$, C, niacinimide and pantothenate in a single solid unit dosage form. The amounts of individual vitamins may vary depending on the estimated safe and adequate daily dietary intakes of particular vitamins. For such multivitamin compositions, a relatively high amount of polyoxyethylene sorbitan ester, *e.g.,* Polysorbate® 80, of approximately 50 to 80% has been found to be effective.

2

As stated above, the vitamin compositions may be prepared in any suitable solid unit dosage form. The solid unit dosage form may be in the form of tablets, hard shell capsules and soft elastic capsules. The increase in bioavailability has been found to be particularly effective with a solid unit dosage form of a soft elastic gelatin capsule. Such a capsule may be manufactured from gelatin, glycerin and optionally sorbitol and is ideal for encapsulation of liquid preparations.

The following examples illustrate vitamin composition formulae. The primary and second pharmaceutical adjuncts are compounded with vitamins and encapsulated into soft elastic gelatin capsules.

### Example 1

| Ingredients | Fill amount per cap |
| --- | --- |
| Vitamin E (as d-alpha Tocopherol) | 500 mg |
| Polysorbate® 80 | 300 mg |
| Ethanol | 50 mg |
| Glycerol | 30 mg |

The bioavailability of vitamin E in the above-mentioned composition was measured and compared to conventional solid dosage forms. The bioavailability was demonstrated to have increased by approximately 27% in humans.

### Example 2

| Ingredients | Fill amount per cap |
| --- | --- |
| Vitamin A (as Vitamin A Palmitate $1 \times 10^6$ i.u./gram) | 50 mg |
| Polysorbate® 80 | 200 mg |
| Glycerol | 20 mg |
| Sorbitan Monooleate | 10 mg |

The bioavailability of vitamin A in the above-mentioned composition was measured and compared with the bioavailability in conventional solid dosage forms. The bioavailability was demonstrated to have increased by approximately 60% in humans.

### Example 3

| Ingredients | Fill amount per cap |
| --- | --- |
| Vitamin A (as Vitamin A Palmitate $1 \times 1^6$ i.u./gram) | 5.0 mg |
| Vitamin D (as Calciferol Solution $2 \times 10^6$ i.u./gram) | 0.2 mg |
| Vitamin E (as d-alpha Tocopherol) | 50.0 mg |
| Vitamin B$_1$ (Thiamine HCl) | 10.0 mg |
| Vitamin B$_2$ (Riboflavin) | 10.0 gm |
| Vitamin B$_6$ (Pyridoxine HCl) | 10.0 mg |
| Vitamin B$_{12}$ (Cyanocobalamin) | 5.0 mg |
| Vitamin C (Ascorbic Acid) | 200.0 mg |
| Niacinamide (Nicotinamide) | 50.0 mg |
| Calcium Pantothenate | 20.0 mg |
| Polysorbate® 80 | 400.0 mg |
| Sorbitan Monooleate | 20.0 mg |

**Claims**

1. A vitamin composition with enhanced bioavailability for incorporation into a solid dosage form comprising a unit dosage amount of at least one vitamin and a primary pharmaceutical adjunct, the vitamin comprising 10 to 80% of the total weight of the composition and the primary pharmaceutical adjunct being a polyoxyethylene sorbitan ester being 30 to 80% of the total weight of the composition, characterised in that the said composition contains a secondary pharmaceutical adjunct consisting of ethanol, glycerol, sorbitol and/or propylene glycol.

2. A composition as claimed in Claim 1 including an oil soluble vitamin A, vitamin D, vitamin E and/or vitamin K.

3. A composition as claimed in Claim 1 or 2 including a water soluble vitamin $B_1$, vitamin $B_2$, vitamin $B_6$, vitamin $B_{12}$, vitamin C, niacinimide and or pantothenic acid.

4. A composition as claimed in Claim 1 or 2 including a single vitamin component said vitamin being an oil soluble vitamin.

5. A composition as claimed in Claim 4 wherein the amount of vitamin used may vary from approximately 15 to 60% of the composition by weight.

6. A composition as claimed in Claim 4 or 5 wherein the ester is present in an amount from approximately 35 to 75% of the composition by weight.

7. A composition as claimed in Claims 1, 2 and 3 incorporating a plurality of vitamins.

8. A composition as claimed in Claim 7 wherein the ester is present in an amount from approximately 50% to 80% of the composition by weight.

9. A composition as claimed in any preceding claim wherein the polyoxyethylene sorbitan ester includes a monooleate.

10. A composition as claimed in any preceding claim wherein the solid unit dosage form is a soft elastic capsule.

**Patentansprüche**

1. Vitaminzusammensetzung mit gesteigerter Bioverfügbarkeit zur Aufnahme in eine feste Dosierungsform mit einer Dosismenge wenigstens eines Vitamins und einem primären pharmazeutischen Zusatz, wobei das Vitamin 10—80% des Gesamtgewichtes der Zusammensetzung und der primäre pharmazeutische Zusatz wie Polyoxyäthylensorbitanester ist, der 30 bis 80% des Gesamtgewichtes der Zusammensetzung ausmacht, dadurch gekennzeichnet, daß die Zusammensetzung einen zweiten pharmazeutischen Zusatz enhält, der aus Äthanol, Glycerin, Sorbit und/oder Propylenglykol besteht.

2. Zusammensetzung nach Anspruch 1, die ein öllösliches Vitamin A, Vitamin D, Vitamin E und/oder Vitamin K enthält.

3. Zusammensetzung nach Anspruch 1 oder 2, die ein wasserlösliches Vitamin $B_1$, Vitamin $B_2$, Vitamin $B_6$, Vitamin $B_{12}$, Vitamin C, Niacinimid und/oder pantothensäure enthält.

4. Zusammensetzung nach Anspruch 1 oder 2, mit einer einzigen Vitaminkomponent, wobei dieses Vitamin ein öllösliches Vitamin ist.

5. Zusammensetzung nach Anspruch 4, wobei die zur Anwendung kommende Vitaminmenge variiert von annähernd 15 bis 60% des Gewichtes der Zusammensetzung.

6. Zusammensetzung nach Anspruch 4 oder 5, wobei der Ester in einer Menge von annähernd 35 bis 75% des Gewichtes der Zusammensetzung enthalten ist.

7. Zusammensetzung nach Anspruch 1, 2 und 3 mit einer Mehrzahl von Vitaminen.

8. Zusammensetzung nach Anspruch 7, wobei der Ester in einer Menge von annähernd 50 bis 80% des Gewichtes der Zusammensetzung enthalten ist.

9. Zusammensetzung nach einem der vorstehenden Ansprüche, wobei der Polyoxyäthylensorbitanester ein Monooleat enthält.

10. Zusammensetzung nach einem der vorstehenden Ansprüche, wobei die feste Dosierungsform eine welche elastische Kapsel ist.

**Revendications**

1. Composition de vitamine(s) ayant une biodisponibilité accue, destinée à être incorporée dans une forme dosée solide, comprenant une quantité, sous forme de doses unitaires, d'au moins une vitamine et d'un produit d'addition, ou excipient, pharmaceutique primaire, la vitamine représentant 10 à 80% du poids total de la composition et l'excipient pharmaceutique primaire étant un ester de polyoxyéthylène sorbitanne représentant 30 à 80% du poids total de la composition, laquelle est caractérisée en ce qu'elle contient un excipient pharmaceutique secondaire consistant en de l'éthanol, du glycérol, du sorbitol et/ou du propylène glycol.

2. Composition telle que revendiquée à la revendication 1, comprenant une vitamine A, une vitamine D, une vitamine E et/ou une vitamine K liposoluble(s).

3. Composition telle que revendiquée à la revendication 1 ou 2, comprenant une vitamine $B_1$, une vitamine $B_2$, une vitamine $B_6$, une vitamine $B_{12}$, une vitamine C, du niacinimide et/ou de l'acide pantothénique hydrosoluble(s).

4. Composition telle que revendiquée à la revendication 1 ou 2, comprenant un seul composant vitaminique, ladite vitamine étant une vitamine liposoluble.

5. Composition telle que revendiquée à la revendication 4, dans laquelle la quantité de la vitamine utilisée peut varier entre environ 15 et 60% du poids de la composition.

6. Composition telle que revendiquée à la revendication 4 ou 5, dans laquelle l'ester est présent en une quantité d'environ 35 à 75% du poids de la composition.

7. Composition telle que revendiquée dans les revendication 1, 2 et 3, incorporant plusieurs vitamines.

8. Composition telle que revendiquée à la revendication 7, dans laquelle l'ester est présent en une quantité représentant environ 50% à 80% du poids de la composition.

9. Composition telle que revendiquée dans l'une quelconque des revendications précédentes, dans laquelle l'ester de polyoxyéthylène sorbitanne est ou comprend un monooléate.

10. Composition telle que revendiquée dans l'une quelconque des revendications précédentes, dans laquelle la forme de dose unitaire solide (ou d'unité posologique solide) est une capsule élastique et molle.